# EUROPEAN PATENT APPLICATION

(11) **EP 2 863 634 A1**
(43) Date of publication of application: **22.04.2015**
(21) Application number: 13803595.1
(22) Date of filing: 15.04.2013
(51) Int. Cl.: H04N 13/04, A61B 1/04, G02B 23/24, G06T 1/00, H04N 7/18

(54) **IMAGE PROCESSING DEVICE AND THREE-DIMENSIONAL IMAGE OBSERVATION SYSTEM**

(30) Priority: 14.06.2012 JP 2012134686
(71) Applicant: Olympus Corporation, Shibuya-ku Tokyo 151-0072 (JP)
(72) Inventor: HATTA, Izumi, Tokyo 151-0072 (JP); NARUSE, Masato, Tokyo 151-0072 (JP); HARAGUCHI, Masafumi, Tokyo 151-0072 (JP); SAKAMOTO, Joji, Tokyo 151-0072 (JP)
(74) Representative: Gunzelmann, Rainer
(86) International application number: PCT/JP2013/061142
(87) International publication number: WO 2013/187116

(57) **Abstract**

Eye fatigue in an observer is reduced in the case in which observation is performed by performing visual comparison between an imaging subject and an auxiliary indication superimposed on the imaging subject in a three-dimensional image. Provided is an image-processing device (2) including a display portion (3) that displays a three-dimensional image reproduced from two parallax images of an imaging subject; attention-point detecting portions (4 and 5) that detect an attention point of the observer observing the three-dimensional image; a parallax calculating portion (6) that calculates parallax between the two parallax images at the detected attention point; an auxiliary-indication creating portion (7) that creates an auxiliary indication to which the same parallax as the parallax calculated by the parallax calculating portion (6) is assigned, that superimposes the created auxiliary indication on the three-dimensional image, and that displays the superimposed image on the display portion (3).

## Description

### {Technical Field}

The present invention relates to an image-processing device and a three-dimensional-image observation system.

### {Background Art}

In the related art, there are known devices that allow an observer to view a three-dimensional image of an imaging subject by using a pair of parallax images obtained by acquiring images of the imaging subject from two points of view that correspond to the left and right eyes (for example, see Patent Literature 1). The device disclosed in Patent Literature 1 is provided with a function for displaying graphic objects such as letters, figures, symbols, and so forth that are added to the three-dimensional image by the observer in a superimposed manner at the same depthwise position as the imaging subject in the three-dimensional image.

### {Citation List}

### {Patent Literature}

{PTL 1} Japanese Unexamined Patent Application, Publication No. 2011-180779

### {Summary of Invention}

### {Technical Problem}

When moving his/her attention point between different depthwise positions in the three-dimensional image, the observer needs to change the angle of convergence formed by lines of sight of the left and right eyes. This adjustment of the angle of convergence causes eye fatigue in the observer. In the case of Patent Literature 1, the angle of convergence is changed every time the attention point is moved between the graphic objects and the imaging subject. Therefore, the device disclosed in Patent Literature 1 is not suitable for use in cases where an imaging subject is observed by performing visual comparison between an auxiliary indication, such as a graphic object, and an imaging subject by frequently moving the attention point back-and-forth therebetween.

The present invention has been conceived in light of the above-described circumstances, and an object thereof is to provide an image-processing device and a three-dimensional-image observation system with which it is possible to reduce eye fatigue in an observer even in the case in which observation is performed by performing visual comparison between an imaging subject and an auxiliary indication by using a three-dimensional image of the imaging subject and the auxiliary indication superimposed on the imaging subject.

### {Solution to Problem}

In order to achieve the above-described object, the present invention provides the following solutions.

A first aspect of the present invention is an image-processing device including a display portion that displays a three-dimensional image of an imaging subject reproduced from two parallax images obtained by acquiring images of the imaging subject; an attention-point detecting portion that detects an attention point of an observer viewing the three-dimensional image displayed on the display portion; a parallax calculating portion that calculates parallax between the two parallax images at the attention point detected by the attention-point detecting portion; and an auxiliary-indication creating portion that creates an auxiliary indication including information about the imaging subject, that superimposes the created auxiliary indication on the three-dimensional image, and that displays the superimposed image on the display portion, wherein the auxiliary-indication creating portion creates the auxiliary indication to which, based on parallax calculated by the parallax calculating portion, the same parallax as the calculated parallax is assigned.

With the first aspect of the present invention, the auxiliary indication created by the auxiliary-indication creating portion is displayed on the display portion in a state in which the auxiliary indication is superimposed on the imaging subject. Therefore, the observer can perform observation by performing visual comparison between the imaging subject and the auxiliary indication in the same three-dimensional image.

In this case, the attention point of the observer in the three-dimensional image displayed on the display portion is detected by the attention-point detecting portion, and the parallax of the imaging subject between the two parallax images at the attention point is calculated by the parallax calculating portion. Then, the auxiliary indication having the same parallax as the parallax at the attention point is created by the auxiliary-indication creating portion and is displayed by being superimposed on the three-dimensional image of the imaging subject. By doing so, the auxiliary indication is displayed in the three-dimensional image at the same depthwise position as the attention point that the observer is currently viewing. Therefore, it is possible to reduce the eye fatigue in the observer even when performing observation by performing visual comparison between the imaging subject and the auxiliary indication.

In the above-described first aspect, the attention-point detecting portion may be provided with a line-of-sight detecting portion that detects lines of sight of left and right eyes of the observer and an attention-point calculating portion that calculates an intersection of the two lines of sight detected by the line-of-sight detecting portion as the attention point.

By doing so, it is possible to precisely detect the attention point of the observer.

In the above-described first aspect, the attention-point detecting portion may detect a target object that the observer observes by using the two parallax images, for example, an instrument for treating a biological object, which serves as the imaging subject, or an affected region that exists in the biological object, and the position of the detected target object may be used as the attention point.

With this configuration, it is possible to simplify the device configuration because the attention point can be detected just by using the image processing.

In the above-described configuration in which the position of the observation target object is detected as the attention point, the attention-point detecting portion may store a reference image obtained by acquiring the target object, and may detect the target object in the parallax images by comparing the reference image with the parallax images.

With this configuration, it is possible to detect the target object in the parallax images by using simple image processing.

In the above-described first aspect, the auxiliary-indication creating portion may display the auxiliary indication at a position at which the auxiliary indication does not overlap with the attention point detected by the attention-point detecting portion in a direction parallel to the plane of the parallax image.

With this configuration, it is possible to prevent the auxiliary indication from interfering with the region being observed by the observer.

In the above-described configuration in which the auxiliary indication is displayed at a position that does not overlap with the attention point, the auxiliary-indication creating portion may set a plurality of predetermined regions as candidates at which to display the auxiliary indication with respect to a display region in the three-dimensional image on the display portion, may select a region having a lower parallax than the parallax at the attention point from the plurality of candidates, and may display the auxiliary indication at the selected region.

With this configuration, of the candidate regions set in advance, because a candidate at which the auxiliary indication would be displayed closer to the front than the imaging subject is selected, it is possible to prevent so-called embedding in which the auxiliary indication is displayed further back than the imaging subject.

In the above-described configuration in which the plurality of candidates are set, priority ranks may be assigned to the plurality of candidates, and the auxiliary-indication creating portion may select a region having the highest priority rank among regions having lower parallax than the parallax at the attention point.

With this configuration, it is possible to display the auxiliary indication in an even more suitable region.

In the above-described first aspect, the auxiliary-indication creating portion may create, as an auxiliary indication, an indication showing a measured value of biometric information of the biological object that serves as the imaging subject, may also set at least two degrees of urgency in accordance with the magnitude of the measured value of the biometric information, may create the auxiliary indication in a normal indication form when the measured value corresponds to the lower degree of urgency, and may create the auxiliary indication in an indication form having a greater emphasis than the normal indication form when the measured value corresponds to the higher degree of urgency.

With this configuration, it is possible to allow the observer to more reliably recognize an abnormality in the measured value of the biometric information, which is important information for the observer.

In the above-described configuration in which the indication form is changed in accordance with the degree of urgency of the measured value of the biometric information, with regard to the auxiliary indication created in the emphasized indication form, the auxiliary-indication creating portion may change the indication form to the normal indication form when the auxiliary indication matches the attention point detected by the attention-point detecting portion.

With this configuration, it is possible to prevent a situation in which the observer is irritated by the use of the emphasized indication that continues even after the observer has confirmed that the degree of urgency is high via the auxiliary indication.

In the above-described first aspect, the auxiliary-indication creating portion may create, as the auxiliary indication, a local-information indication including information about a specific position in the imaging subject, may display the auxiliary indication on the display portion, and may move the auxiliary indication in the direction of the plane based on a track of the attention point obtained by the attention-point detecting portion.

With this configuration, when the viewing field of the parallax images is changed and the target object is moved in the three-dimensional image, the auxiliary indication is also moved to follow the target object. Therefore, it is possible to easily make the observer recognize the correspondence relationship between the target object and the auxiliary indication.

In the above-described first aspect, the attention-point detecting portion may obtain a track of the attention point by storing the position of the detected attention point over time, and the auxiliary-indication creating portion may create, as the auxiliary indication, a local-information indication including information about a specific position in the imaging subject and an arrow that points to the specific position from the local-information indication, may assign the same parallax as a parallax at the attention point to the local-information indication and a base of the arrow, and may assign the same parallax as a parallax at the specific position to a tip of the arrow.

With this configuration, when the observer is observing a position that differs from the specific position in the depth direction, the local-information indication is displayed at a position that differs from the specific position in the depth direction. In such a situation also, it is possible to easily make the observer recognize the correspondence relationship between the local-information indication and the specific position by means of the arrow that also extends in the depth direction.

A second aspect of the present invention is a three-dimensional-image observation system including an image-acquisition device that obtains two parallax images by acquiring images of an imaging subject; and any one of image-processing device described above.

A third aspect of the present invention is a three-dimensional-image observation system including an image-acquisition device that obtains two parallax images by acquiring images of an imaging subject; and an image-processing device described above, wherein the image-acquisition device obtains, as the parallax images, a normal image acquired by irradiating the imaging subject with illumination light and a fluorescence image obtained by acquiring, by irradiating the imaging subject with excitation light, fluorescence from a fluorescent substance provided in the target object, and the attention-point detecting portion detects, as the target object, a fluorescence region in the fluorescence image.

With this configuration, it is possible to detect the target object in the fluorescence image, which is a parallax image, by using simple image processing.

### {Advantageous Effects of Invention}

With the present invention, an advantage is afforded in that it is possible to reduce eye fatigue in an observer even in the case in which observation is performed by performing visual comparison between an imaging subject and an auxiliary indication by using a three-dimensional image of the imaging subject and the auxiliary indication superimposed on the imaging subject.

### {Brief Description of Drawings}

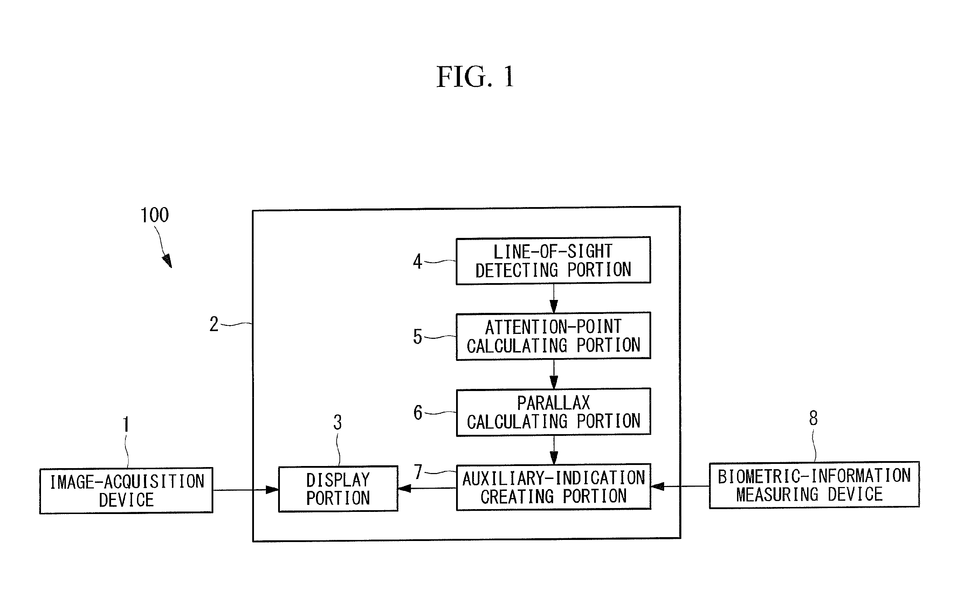

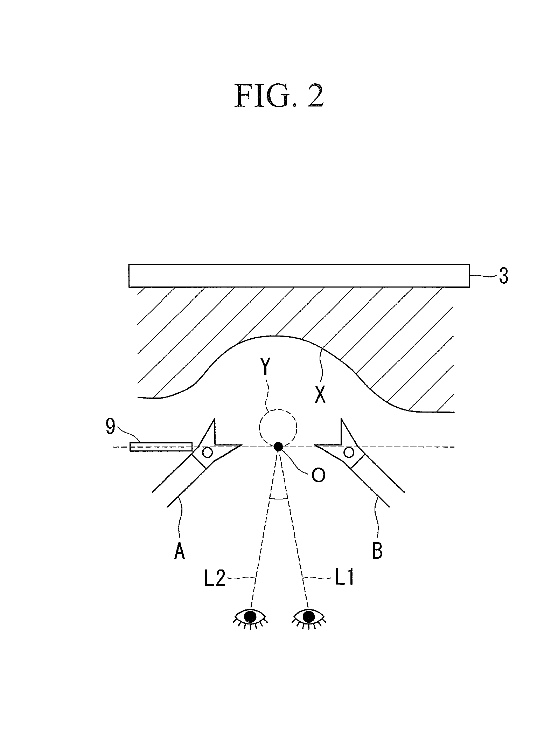

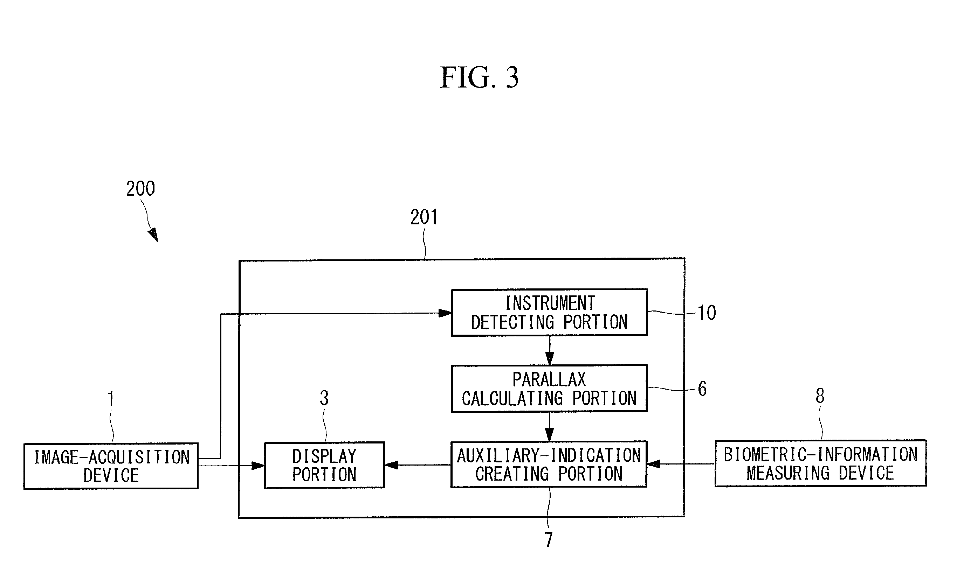

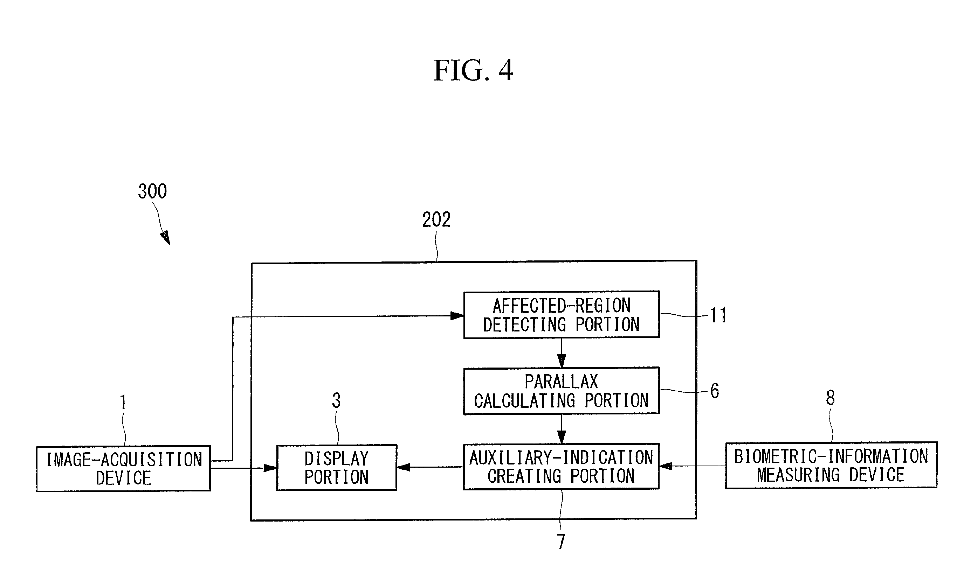

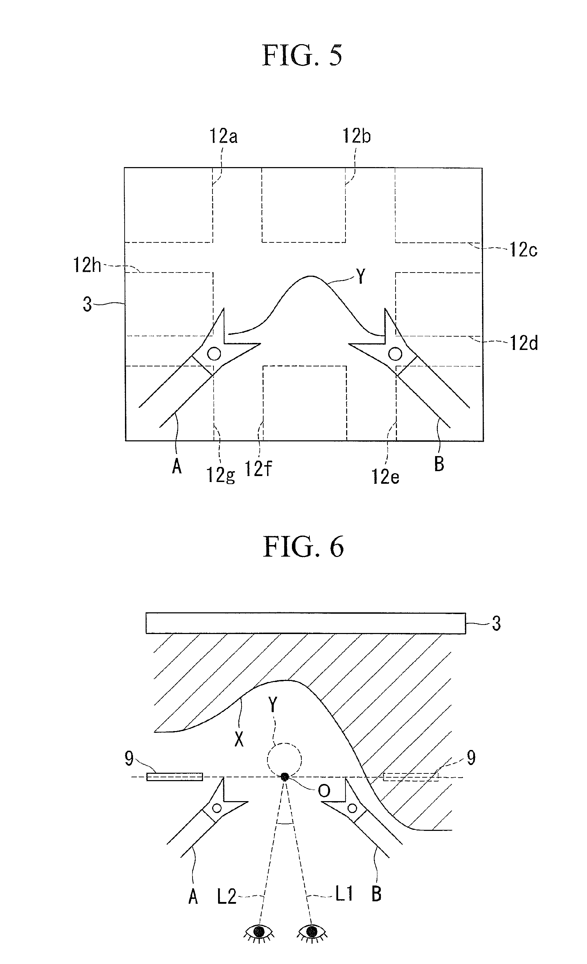

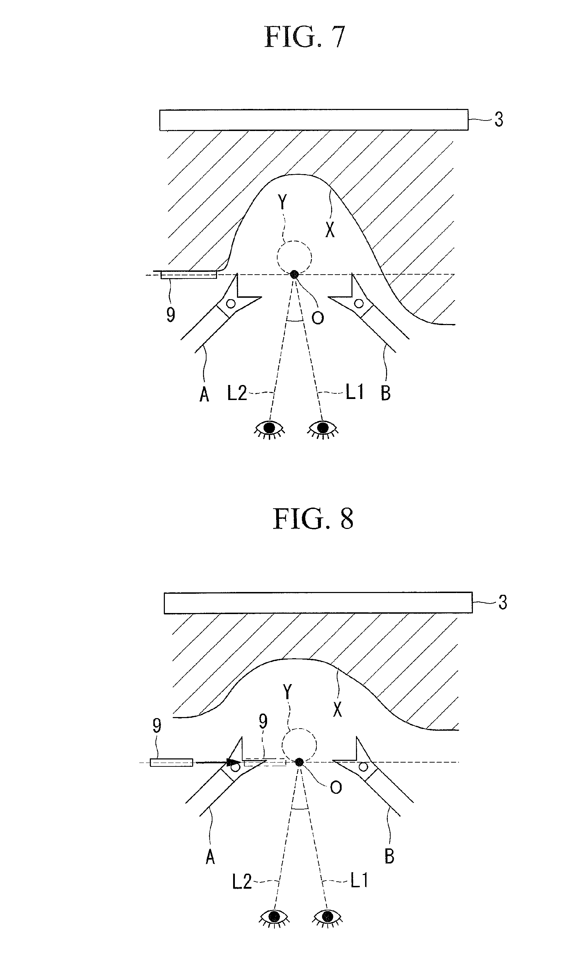

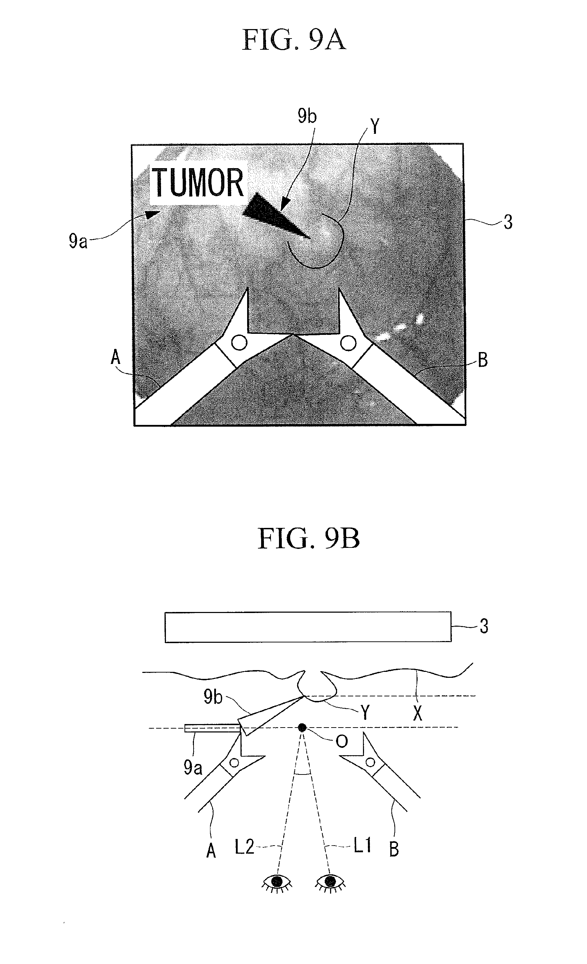

{Fig. 1} Fig. 1 is a diagram showing the overall configuration of a three-dimensional-image observation system according to an embodiment of the present invention.
{Fig. 2} Fig. 2 is a diagram showing a three-dimensional image reproduced by the three-dimensional-image observation system in Fig. 1, as seen from above a display portion.
{Fig. 3} Fig. 3 is a diagram showing the overall configuration of the three-dimensional-image observation system according to a first modification.
{Fig. 4} Fig. 4 is a diagram showing the overall configuration of the three-dimensional-image observation system according to a second modification.
{Fig. 5} Fig. 5 is a diagram showing candidate regions at which to display an auxiliary indication in a three-dimensional-image observation system according to a third modification.
{Fig. 6} Fig. 6 is a diagram showing a three-dimensional image reproduced by the three-dimensional-image observation system according to the third modification, as seen from above a display portion.
{Fig. 7} Fig. 7 is a diagram showing another three-dimensional image reproduced by the three-dimensional-image observation system according to the third modification, as seen from above the display portion.
{Fig. 8} Fig. 8 is a diagram showing a three-dimensional image reproduced by a three-dimensional-image observation system according to a fourth modification, as seen from above a display portion.
{Fig. 9A} Fig. 9A is a diagram showing a three-dimensional image reproduced by a three-dimensional-image observation system according to a fifth modification, as seen from the front of a display portion.
{Fig. 9B} Fig. 9B is a three-dimensional image reproduced by the three-dimensional-image observation system according to the fifth modification, as seen from above the display portion.

### {Description of Embodiment}

A three-dimensional-image observation system 100 according to an embodiment of the present invention will be described below with reference to the drawings.

As shown in Fig. 1, the three-dimensional-image observation system 100 according to this embodiment is provided with an image-acquisition device 1 that acquires an image of an imaging subject and an image-processing device 2 that reproduces and displays a three-dimensional image of the imaging subject based on two two-dimensional images of the imaging subject obtained by the image-acquisition device 1.

The image-acquisition device 1 is, for example, an endoscope (hereinafter, also referred to as the endoscope 1) for observing, as the imaging subject, tissue inside the body of a subject, and has two objective lenses (not shown) that correspond to the right eye and the left eye of an observer. Two two-dimensional images acquired by the image-acquisition device 1 by using the two objective lenses are parallax images in which the imaging subject is observed from different points of view. This pair of parallax images constitute parallax images needed to reproduce a three-dimensional image of the imaging subject.

Note that, with the image-acquisition device 1, the pair of parallax images may be created by using a pupil-division system instead of creating the pair of parallax images by using the two objective lenses. Specifically, the image-acquisition device 1 may be provided with a single objective lens that forms an optical image of the imaging subject and a pupil-division portion, such as a prism, that divides the optical image of the imaging subject formed by the objective lens into left and right parts centered on the optical axis, and a pair of parallax images may be obtained by individually acquiring the two optical images divided by the pupil-division portion.

The image-processing device 2 is provided with a display portion 3 that receives the parallax images from the image-acquisition device 1 and displays the parallax images as a three-dimensional image; a line-of-sight detecting portion (attention-point detecting portion) 4 that detects the lines of sight of the left and right eyes of the observer viewing the display portion 3; an attention-point calculating portion (attention-point detecting portion) 5 that calculates an attention point at which the left and right lines of sight detected by the line-of-sight detecting portion 4 intersect each other; a parallax calculating portion 6 that calculates the parallax of the parallax images at the attention point detected by the attention-point calculating portion 5; and an auxiliary-indication creating portion 7 that creates an auxiliary indication based on information about the imaging subject, that superimposes the auxiliary indication on the three-dimensional image of the imaging subject, and that displays the superimposed image on the display portion 3.

The line-of-sight detecting portion 4 detects positions of the left and right eyeballs of the observer facing the display portion 3 and calculates the left and right lines of sight of the observer based on the detected eyeball positions. As a means of detecting the eyeball positions, for example, a camera that is provided in the display portion 3 and that acquires images of the left and right eyeballs of the observer is employed. The eyeball positions may be detected by using means other than a camera.

As an attention point to which the observer is currently paying attention, the attention-point calculating portion 5 calculates a position at which the left and right lines of sight of the observer detected by the line-of-sight detecting portion 4 intersect each other.

The parallax calculating portion 6 calculates the parallax between the left and right parallax images at the attention point calculated by the attention-point calculating portion 5.

The auxiliary-indication creating portion 7 receives measured values from a biometric-information measuring device 8 that measures biometric information (for example, heart rate, blood pressure, and body temperature) of the subject, and creates an auxiliary indication that shows the received measured values. Then, the auxiliary-indication creating portion 7 creates left and right auxiliary-indication parallax images that display the auxiliary indications. At this time, the auxiliary-indication creating portion 7 sets the parallax between the auxiliary indication in the right auxiliary-indication parallax image and the auxiliary indication in the left auxiliary-indication parallax image so as to be equal to the parallax calculated by the parallax calculating portion 6. The auxiliary-indication creating portion 7 outputs the created left and right auxiliary-indication parallax images to the display portion 3.

Note that, in addition to the indications showing the measured values of the biometric-information of the subject, the auxiliary-indication creating portion 7 may create other indications showing other details as auxiliary indications 9. For example, as the auxiliary indications 9, the image-processing device 2 may create a letter sequence, a grid, and a scale that shows the size of the imaging subject, which are arbitrarily input by the observer, an image of the imaging subject acquired from another angle by another image-acquisition device, an image that serves as a diagnostic reference, and so forth and may display them on the display portion 3.

The display portion 3 individually superimposes the left and right auxiliary-indication parallax images received from the auxiliary-indication creating portion 7 on the left and right parallax images received from the image-acquisition device 1 and constructs a three-dimensional image by using the superimposed parallax images.

Next, the operation of the thus-configured three-dimensional-image observation system 100 will be described.

With the three-dimensional-image observation system 100 according to this embodiment, tissue inside the body of a subject is observed by using the endoscope 1 that serves as the image-acquisition device, and the obtained images are displayed to the observer in the form of a three-dimensional image.

First, the endoscope 1 obtains a pair of parallax images with different points of view by acquiring images of the tissue inside the body of the subject at the same time by using the left and right objective lenses. The obtained pair of parallax images are successively transmitted to the image-processing device 2 and are converted to a three-dimensional image which is subsequently displayed on the display portion 3.

Meanwhile, the biometric information of the subject is measured by the biometric-information measuring device 8, and the measured values thereof are transmitted to the image-processing device 2. The image-processing device 2 creates auxiliary indications showing the measured values of the individual pieces of biometric information received from the biometric-information measuring device 8, and displays these auxiliary indications superimposed on the three-dimensional image of the tissue inside the body. By doing so, the observer can check the tissue inside the body of the subject and the measured values of the biometric information on the same three-dimensional image.

Here, as shown in Fig. 2, based on an attention point O in the three-dimensional image, to which the observer is currently paying attention, the image-processing device 2 displays the auxiliary indication 9 at a position at which the parallax thereof would be equal to the parallax at the attention point O. Specifically, lines of sight L1 and L2 of the observer who is observing the three-dimensional image displayed on the display portion 3 are detected by the line-of-sight detecting portion 4, and the position of the attention point O to which the observer is currently paying attention is calculated by the attention-point calculating portion 5 based on the detected lines of sight L1 and L2. Then, the parallax between the left and right parallax images at the attention point O is calculated by the parallax calculating portion 6, and the auxiliary-indication creating portion 7 creates auxiliary-indication parallax images in which the auxiliary indication 9 is displayed at the position at which the parallax thereof is equal to the calculated parallax.

The display portion 3 superimposes the right auxiliary-indication parallax image on the right parallax image, superimposes the left auxiliary-indication parallax image on the left parallax image, and constructs a three-dimensional image by using the left and right superimposed parallax images. By doing so, the auxiliary indication 9 is displayed at the same depthwise position as that of the attention point O to which the observer is currently paying attention. In the figures, reference signs A and B indicate instruments operated by the observer, reference sign X indicates tissue inside the body, and reference sign Y indicates an affected region.

As has been described above, with this embodiment, the attention point O is detected based on the lines of sight L1 and L2 of the observer, and the auxiliary indication 9 is displayed at the same depthwise position as this attention point O. Therefore, the observer can view the auxiliary indication 9 by moving the lines of sight L1 and L2 from the attention point O without changing the angle of convergence. Accordingly, there is an advantage in that eye fatigue can be reduced even in the case in which the tissue X or affected region Y is observed while visually checking the auxiliary indication 9 frequently.

Here, if the auxiliary indication 9 is moved in the depth direction so as to successively follow fine movement of the lines of sight L1 and L2 of the observer, this frequent movement of the auxiliary indication 9 could irritate the observer. Therefore, the auxiliary indication 9 may be allowed to move so as to follow the attention point O only when the attention point O of the observer remains at substantially the same position for a certain amount of time. By doing so, the auxiliary indication 9 is prevented from moving more than necessary, thus reducing the irritation experienced by the observer.

Specifically, the parallax calculating portion 6 stores the parallax calculated at the attention point O over time, starts a clock when the parallax changes due to a movement of the attention point O, and, in the case in which a predetermined amount of time has passed, newly outputs the parallax at the moved attention point O to the auxiliary-indication creating portion 7. The auxiliary-indication creating portion 7 stores the parallax received from the parallax calculating portion 6, and continues to create the auxiliary-indication parallax images by using the stored parallax. Then, in the case in which a new parallax is received from the parallax calculating portion 6, the auxiliary-indication creating portion 7 updates the stored parallax to the new parallax, and creates the auxiliary-indication parallax images by using this new parallax. Accordingly, the depthwise position of the auxiliary indication 9 displayed on the three-dimensional image is changed when the attention point O of the observer remains at substantially the same position for a certain amount of time.

Note that, instead of measuring the time after the attention point O has moved, the parallax calculating portion 6 may calculate an average parallax at the attention point O within a predetermined window of time, and may output the calculated average parallax to the auxiliary-indication creating portion 7.

By doing so, the auxiliary indication 9 is made less sensitive to following the fine movement of the attention point O of the observer, and the auxiliary indication 9 is kept sufficiently sensitive to following coarse movement of the attention point O of the observer. In this way also, the auxiliary indication 9 can be prevented from moving more than necessary, thus eliminating the irritation experienced by the observer.

Next, modifications of the above-described three-dimensional-image observation system 100 will be described.

### (First Modification)

A three-dimensional-image observation system 200 according to a first modification is configured so that an image-processing device 201 uses instruments (target objects) A and B in images obtained by the image-acquisition device 1 as the attention point O of the observer.

In this modification, as shown in Fig. 3, the image-processing device 201 is provided with an instrument detecting portion (attention-point detecting portion) 10 that detects instruments A and B that are operated by the observer in the parallax images and that follows the detected instruments A and B. The instrument detecting portion 10 stores images of the instruments A and B to be used by the operator as reference images, and searches the parallax images for regions that match the reference images or that are similar thereto. Then, as the attention point O of the observer, the instrument detecting portion 10 calculates the centers of gravity of the qualifying regions. Processing by the parallax calculating portion 6 and the auxiliary-indication creating portion 7 after detecting the attention point O are as have been described above.

With the thus-configured three-dimensional-image observation system 200 according to this modification, when treating the affected region Y by using the instruments A and B, the operator pays attention to the instruments A and B or the vicinity of the instruments A and B. Therefore, also by detecting the instruments A and B as the attention point O, it is possible to detect the attention point O of the observer with sufficiently high precision. In addition, because the attention point O is detected just by using the image processing, the device configuration can be simplified as compared with the configuration in which the attention point O is calculated by detecting the lines of sight L1 and L2 of the observer.

In this modification, the instrument detecting portion 10 may detect the instruments A and B in the parallax images received from the endoscope 1 by detecting an identification substance provided in the instruments A and B.

For example, fluorescent paint may be applied to at least a portion of the instruments A and B, and the instruments A and B may be detected in the fluorescence images obtained by acquiring images of fluorescence emitted from the fluorescent paint. It is preferable to use fluorescent paint that is excited by UV light or IR light so that white-light images of the tissue X do not include the fluorescence.

By irradiating the tissue X inside the body in a time-division manner with white light (illumination light) to obtain white-light images (normal images) and with UV light or IR light to obtain fluorescence images, the endoscope 1 obtains the white-light images and the fluorescence images in a time-division manner. Alternatively, the endoscope 1 irradiates the tissue X inside the body with the white light and the IR light or the UV light at the same time, white light and fluorescence are separated by using a wavelength separation filter or the like, and white-light images and fluorescence images are obtained at the same time by detecting the white light and the fluorescence separately.

The instrument detecting portion 10 receives the fluorescence images that serve as the parallax images from the endoscope 1, identifies, as the instruments A and B, regions in the fluorescence images having higher luminance than a predetermined threshold, and calculates positions of the centers of gravity of the identified regions as the attention point O.

By doing so also, the attention point O of the observer can be detected with sufficiently high precision while simplifying the device configuration.

### (Second Modification)

A three-dimensional-image observation system 300 according to a second modification is configured so that an image-processing device 202 uses an affected region (target object) Y in images obtained by the image-acquisition device 1 as the attention point O of the observer.

In this modification, as shown in Fig. 4, the image-processing device 2 is provided with an affected-region detecting portion (attention-point detecting portion) 11 that identifies the affected region Y in the parallax images received from the endoscope 1 and that calculates the center of gravity of the identified affected region Y as the attention point O. The affected-region detecting portion 11 stores images obtained by acquiring images of various types of affected region as reference images, searches the parallax images for regions that match the individual reference images or that are similar thereto, and calculates the centers of gravity of the matching regions as the attention points O.

After identifying the affected regions Y, with regard to the identified affected regions Y, the affected-region detecting portion 11 follows the affected regions Y by continuing to match the previously-matched reference images and the affected regions Y, and obtains tracks of the attention points O by storing the positions of the centers of gravity of the affected regions Y over time.

In this modification, the auxiliary-indication creating portion 7 creates information about the individual affected regions Y as the auxiliary indications 9 and displays the auxiliary indications 9 for these affected regions Y in the vicinities of the affected regions Y so that the auxiliary indications 9 follow the movement of the affected regions Y in the three-dimensional image.

Specifically, the auxiliary-indication creating portion 7 receives from the affected-region detecting portion 11 a signal indicating that an affected region Y has been found, creates the auxiliary indication (local-information indication) 9 by prompting the observer to input a letter sequence which serves as the information about the affected region Y, and, in addition prompts the observer to specify a region at which to display the created auxiliary indication 9. Inputting the letter sequence and specifying the display region by the operator are, for example, performed by using a Graphical User Interface (GUI) provided in the auxiliary-indication creating portion 7. Then, the auxiliary-indication creating portion 7 receives the track of the attention point O from the affected-region detecting portion 11, and causes the auxiliary indication 9 to be moved along the same track as the attention point O. By doing so, the auxiliary indication 9 is moved in the three-dimensional image while maintaining a certain positional relationship with the affected region Y.

With the thus-configured three-dimensional-image observation system 300 according to this modification, when an affected region Y exists in the three-dimensional image, the operator pays attention to the affected region Y. Therefore, also by detecting the affected region Y as the attention point O, it is possible to detect the attention point O of the observer with sufficiently high precision. In addition, because the attention point O is detected just by using the image processing, the device configuration can be simplified as compared with the configuration in which the attention point O is calculated by detecting the lines of sight L1 and L2 of the observer. Furthermore, when the endoscope 1 is moved inside the body and the viewing field is changed, the auxiliary indication 9 as well as the corresponding affected region Y are moved along with the movement of the viewing field. Therefore, the observer can easily recognize the correspondence relationship between the auxiliary indication 9 and the affected region Y.

In this modification, in the case in which the auxiliary indication 9 is displayed at an inappropriate position as a result of the movement of the auxiliary indication 9, the image-processing device 2 may issue a warning to that effect. For example, a warning is displayed on the three-dimensional image in the case in which the auxiliary indication 9 falls outside the display area of the display portion 3 or in the case in which the auxiliary indication 9 is displayed at a position that overlaps with another affected region Y. After issuing the warning, the auxiliary-indication creating portion 7 prompts the observer to re-specify the position of the region at which to display the auxiliary indication 9 and displays it at the re-specified display position.

### (Third Modification)

As shown in Fig. 5, a three-dimensional-image observation system according to a third modification is configured so that the auxiliary-indication creating portion 7 selects one of candidates 12a to 12h for the region at which to display the auxiliary indication 9 and displays the auxiliary indication 9 in the selected region among the candidates 12a to 12h.

In this modification, the auxiliary-indication creating portion 7 stores a plurality of candidates 12a to 12h for the region at which to display the auxiliary indication 9. Because the observer normally performs observation by placing the observation-target region at substantially the center of the three-dimensional image, it is preferable that regions serving as the candidates 12a to 12h be set at regions at peripheral portions of the parallax images so as not to overlap with the observation-target region used by the observer.

The auxiliary-indication creating portion 7 stores the plurality of candidates 12a to 12h with priority ranks assigned thereto. Then, the auxiliary-indication creating portion 7 selects the candidate having the highest priority rank first, for example, the candidate 12a, calculates the parallax between the left and right parallax images at the position of the candidate 12a, and compares the calculated parallax with the parallax at the attention point O calculated by the parallax calculating portion 6. Then, if the parallax at the position of the candidate 12a is less than the parallax at the attention point O, the auxiliary-indication creating portion 7 displays the auxiliary indication 9 in the region of this candidate 12a.

On the other hand, if the parallax at the position of the candidate 12a is greater than the parallax at the attention point O, the auxiliary-indication creating portion 7 selects the second-ranking candidate, for example, the candidate 12b, and similarly compares the parallax at the position of the second-ranking candidate 12b with the parallax at the attention point O. Subsequently, the auxiliary-indication creating portion 7 repeats the same processing until a candidate for which the parallax at the position of the candidate is equal to or less than the parallax at the attention point 0 is found. By doing so, of the candidates 12a to 12h, a candidate at which the auxiliary indication 9 will be displayed at a position closer to the front than the tissue X is determined.

With the thus-configured three-dimensional-image observation system according to this modification, as shown in Fig. 6, the auxiliary indication 9 is displayed at a position closer to the front than the tissue X. By doing so, it is possible to prevent the occurrence of so-called embedding in which the auxiliary indication 9 is displayed at a position further back than the tissue X in the three-dimensional image (see broken lines in the figure).

In this modification, the auxiliary-indication creating portion 7 may be configured so as to prompt the observer to specify, of the plurality of candidates 12a to 12h, the candidate at which the auxiliary indication 9 will be displayed by using means such as a GUI or the like. In this case, the observer selects the candidate with which embedding does not occur based on the shape of the tissue X in the three-dimensional image.

In addition, in this modification, the auxiliary-indication creating portion 7 may store predetermined regions (for example, peripheral portions, lower portions, or the like of the parallax images) at which to display the auxiliary indication 9 instead of the plurality of candidates 12a to 12h, may select, from among the stored regions, a position at which the parallax would be smaller than the parallax at the attention point O, and may display the auxiliary indication 9 at the selected position. By doing so also, it is possible to prevent embedding of the auxiliary indication 9 as with the case in which one region is selected from the plurality of candidates 12a to 12h.

In addition, in this modification, predetermined regions at which to display the auxiliary indication 9 may be set, the display portion 3 may edit a portion of the three-dimensional image of the tissue X so that the tissue X would be displayed further back than the auxiliary indication 9 in the predetermined regions, and thus, embedding of the auxiliary indication 9 may be prevented in this way.

In this case, the display portion 3 stores the predetermined regions at which to display the auxiliary indication 9, and assigns the same parallax as the parallax at the attention point O to regions in the parallax images that match the predetermined regions in the case in which the parallax at these regions is greater than the parallax at the attention point O. By doing so, a portion of the three-dimensional image is flattened, as shown in Fig. 7.

### (Fourth Modification)

A three-dimensional-image observation system according to a fourth modification is configured so that, a plurality of degrees of urgency are set depending on the magnitude of values of the individual pieces of biometric information, and the auxiliary-indication creating portion 7 changes the indication form in accordance with the degree of urgency.

In this modification, in the case in which a measured value received from the biometric-information measuring device 8 corresponds to a lower degree of urgency, the auxiliary-indication creating portion 7 creates the auxiliary indication 9 in a normal indication form. On the other hand, in the case in which a measured value received from the biometric-information measuring device 8 corresponds to a higher degree of urgency, the auxiliary-indication creating portion 7 creates the auxiliary indication 9 in an indication form having a greater emphasis than the normal indication form. As the emphasized indication form, for example, color changes, enlarged display, blinking, rotation, vibration, or the like is employed. In addition, the degrees of urgency can be set in arbitrary steps.

With the thus-configured three-dimensional-image observation system according to this modification, in the case in which the degree of urgency is increased when an abnormality appears in the biometric information, such as heart rate, blood pressure, or the like, the auxiliary indication 9 for that biometric information will be displayed with emphasis in the three-dimensional image. By doing so, there is an advantage in that it is possible to quickly and reliably make the observer recognize the abnormality in the subject, which is important information for the observer.

In this modification, the auxiliary-indication creating portion 7 may be configured so as to return the emphasized indication form to the normal indication form after confirming that the observer has paid attention to the emphasized information indication. Specifically, the auxiliary-indication creating portion 7 receives the calculated position of the attention point O from the attention-point calculating portion 5, and returns the auxiliary indication 9 to the normal indication form if the position of the attention point O matches the display region of the auxiliary indication 9. By doing so, it is possible to prevent a situation in which the observer is irritated by continuing to unnecessarily show the auxiliary indication 9 in an emphasized manner even after the observer has confirmed the abnormality in the subject.

In addition, in this modification, the auxiliary-indication creating portion 7 may be configured so that, in the case in which a measured value of the biometric information received from the biometric-information measuring device 8 corresponds to a higher degree of urgency, the auxiliary indication 9 is moved so as to follow the attention point O of the observer.

Specifically, in the case in which a measured value received from the biometric-information measuring device 8 corresponds to a lower degree of urgency, the auxiliary-indication creating portion 7 displays the auxiliary indication 9 in a predetermined display region. Also, in the case in which the degree of urgency of the measured value received from the biometric-information measuring device 8 is increased, the auxiliary-indication creating portion 7 receives the calculated position of the attention point O from the attention-point calculating portion 5, and displays the auxiliary indication 9 in the vicinity of the position of the attention point O.

By doing so, as shown in Fig. 8, the auxiliary indication 9 is moved, for example, from a peripheral portion of the three-dimensional image to the center portion of the three-dimensional image to which the observer is paying attention. Then, the auxiliary-indication creating portion 7 calculates the amount of movement of the attention point O, and causes the auxiliary indication 9 to follow the movement of the attention point O by adding the calculated amount of movement to the auxiliary indication 9 also.

In this way, it is also possible to reliably make the observer recognize the auxiliary indication 9 by forcedly inserting the auxiliary indication 9 containing important information for the observer into the region to which the observer is paying attention.

The auxiliary indication 9 following the movement of the attention point O may be stopped after continuing to do so for a predetermined amount of time or may be stopped when it is confirmed that the observer has paid attention to the auxiliary indication 9, as described above. After the auxiliary indication 9 following the movement of the attention point O is stopped, the auxiliary indication 9 may be returned to the normal display region or may be deleted. In the case in which the auxiliary indication 9 is deleted, in order to make the observer recognize the auxiliary indication 9 even more strongly, the auxiliary indication 9 may be deleted gradually.

### (Fifth Modification)

A three-dimensional-image observation system according to a fifth modification is configured so that the auxiliary-indication creating portion 7 creates, as the auxiliary indications 9, a local-information indication about a specific position in the tissue X and an arrow that indicates the specific position indicated by the local-information indication.

In this modification, in the case in which an affected region Y exists in the parallax images, the auxiliary-indication creating portion 7 creates, as the auxiliary indications 9, a letter sequence (local-information indication) about that affected region Y and an arrow that points at the affected region Y. For example, as shown in Fig. 9A, in the case in which the affected region Y is a tumor, the auxiliary-indication creating portion 7 creates a letter sequence 9a that reads "tumor" and an arrow 9b that points at the position of the tumor Y indicated by the letter sequence 9a. The letter sequence 9a is input by the observer by, for example, using a keyboard. Positions of a starting point (base of the arrow 9b) and an ending point (tip of the arrow 9b) of the arrow 9b in the direction of a plane are specified by the observer by, for example, using a GUI provided in the auxiliary-indication creating portion 7.

Here, the auxiliary-indication creating portion 7 assigns the same parallax as the letter sequence 9a to the starting point of the arrow 9b and the same parallax as the tumor Y serving as the specific position to the ending point of the arrow 9b. Then, as shown in Fig. 9B, the auxiliary-indication creating portion 7 creates the arrow 9b in which the parallax gradually changes from the starting point toward the ending point.

Because the letter sequence 9a is displayed at the same depthwise position as the tumor Y in the three-dimensional image when the observer is paying attention to the tumor Y, it is easy to recognize that the letter sequence 9a corresponds to the tumor Y. However, when the observer is paying attention to a position other than the tumor Y, it is difficult to recognize that the letter sequence 9a corresponds to the tumor Y partly because the letter sequence 9a and the tumor Y are displayed at different depthwise positions. In particular, in the case in which there are a plurality of letter sequences 9a in the three-dimensional image, it is difficult to grasp the correspondence relationship between the letter sequences 9a and the specific positions at a glance.

Therefore, with the three-dimensional-image observation system according to this modification, by displaying the arrows 9b that extend in the depth direction from the letter sequences 9a toward the specific positions, the observer can easily recognize the correspondence between the respective letter sequences 9a and specific positions.

In this modification, the auxiliary-indication creating portion 7 may be configured so as to set the starting point and the ending point of the arrow 9b.

In order to distinguish the affected region Y existing in the tissue X inside the body, special-light observation is sometimes employed by using the endoscope 1. The image-processing device 2 is provided with an affected-region identifying portion (not shown) that receives a special-light image from the endoscope 1 and that identifies an affected region in the special-light image.

The endoscope 1 has a normal mode for obtaining a white-light image of the tissue X inside the body and a special mode for obtaining a special-light image of the tissue X inside the body, and the normal mode and the special mode are switched when the observer switches a switch (not shown). The special-light image is, for example, a fluorescence image, a narrowband-light image, or an IR-light image. In the special-light image, the affected region is observed to have a different luminance value or hue than the peripheral portions.

As an affected region, the affected-region identifying portion identifies a region having a higher luminance value than the peripheral portions or a region having different hue than the peripheral portions. Then, the auxiliary-indication creating portion 7 sets the starting point of the arrow 9b at an end of the region in which the letter sequence 9a is to be displayed and sets the ending point of the arrow 9b at the center of the affected region identified by the affected-region identifying portion.

With the thus-configured three-dimensional-image observation system, the observer checks the presence/absence of the affected region by using the special mode. In the case in which the presence of the affected region is confirmed in the special-light image, that affected region is identified by the affected-region identifying portion, the arrow 9b indicating the affected region is created by the auxiliary-indication creating portion 7, and the arrow 9b is displayed in the three-dimensional image. This arrow 9b continues to be displayed in the three-dimensional image based on the white-light image even after the observer switches the operation to the normal mode. By doing so, operations performed by the observer to specify the starting point and the ending point of the arrow 9b are eliminated, thus making it possible to reduce the burden on the observer.

In addition, in this modification, the auxiliary-indication creating portion 7 may switch between display and non-display of the auxiliary indication 9 in accordance with the attention point O of the observer.

In this case, the auxiliary-indication creating portion 7 receives the calculated position of the attention point O from the attention-point calculating portion 5, and, in the case in which the position of the attention point O matches the position of the affected region Y, displays the letter sequence 9a about the affected region Y and the arrow 9b in the auxiliary-indication parallax images. On the other hand, in the case in which the position of the attention point O deviates from the position of the affected region Y, the letter sequence 9a about the affected region Y and the arrow 9b are deleted from the auxiliary-indication parallax images.

By doing so, of the auxiliary indications 9 related to the specific positions, the auxiliary indication 9 related to the specific position to which the observer is currently paying attention is displayed on the three-dimensional image, and the auxiliary indications 9 that the observer does not currently need are deleted from the three-dimensional image. Accordingly, it is possible to prevent the three-dimensional image from becoming unnecessarily complicated.

### {Reference Signs List}

1 image-acquisition device
2 image-processing device
3 display portion
4 line-of-sight detecting portion (attention-point detecting portion)
5 attention-point calculating portion (attention-point detecting portion)
6 parallax calculating portion
7 auxiliary-indication creating portion
8 biometric-information measuring device
9 auxiliary indication
10 instrument detecting portion (attention-point detecting portion)
11 affected-region detecting portion (attention-point detecting portion)
12a to 12h candidate
100 three-dimensional-image observation system
A, B instrument
L1, L2 line of sight
O attention point
X tissue (imaging subject)
Y affected region

## Claims

1. An image-processing device comprising:
a display portion that displays a three-dimensional image of an imaging subject reproduced from two parallax images obtained by acquiring images of the imaging subject;
an attention-point detecting portion that detects an attention point of an observer viewing the three-dimensional image displayed on the display portion;
a parallax calculating portion that calculates parallax between the two parallax images at the attention point detected by the attention-point detecting portion; and
an auxiliary-indication creating portion that creates an auxiliary indication including information about the imaging subject, that superimposes the created auxiliary indication on the three-dimensional image, and that displays the superimposed image on the display portion,
wherein the auxiliary-indication creating portion creates the auxiliary indication to which, based on parallax calculated by the parallax calculating portion, the same parallax as the calculated parallax is assigned.

2. The image-processing device according to Claim 1, wherein the attention-point detecting portion is provided with a line-of-sight detecting portion that detects lines of sight of left and right eyes of the observer and an attention-point calculating portion that calculates an intersection of the two lines of sight detected by the line-of-sight detecting portion as the attention point.

3. The image-processing device according to Claim 1, wherein the attention-point detecting portion detects a target object that the observer observes from the two parallax images, and uses a position of the detected target object as the attention point.

4. The image-processing device according to Claim 3, wherein the attention-point detecting portion stores a reference image obtained by acquiring the target object, and detects the target object in the parallax images by comparing the reference image with the parallax images.

5. The image-processing device according to Claim 3 or 4, wherein the target object is an instrument for treating a biological object, which serves as the imaging subject.

6. The image-processing device according to Claim 3 or 4, wherein the target object is an affected region that exists in a biological object, which serves as the imaging subject.

7. The image-processing device according to any one of Claims 1 to 6, wherein the auxiliary-indication creating portion displays the auxiliary indication at a position at which the auxiliary indication does not overlap with the attention point detected by the attention-point detecting portion in a direction parallel to the plane of the parallax image.

8. The image-processing device according to Claim 7, wherein the auxiliary-indication creating portion sets a plurality of predetermined regions as candidates at which to display the auxiliary indication with respect to a display region in the three-dimensional image on the display portion, selects a region having a lower parallax than the parallax at the attention point from the plurality of candidates, and displays the auxiliary indication at the selected region.

9. The image-processing device according to Claim 8, wherein priority ranks are assigned to the plurality of candidates, and the auxiliary-indication creating portion selects a region having the highest priority rank among regions having lower parallax than the parallax at the attention point.

10. The image-processing device according to any one of Claims 1 to 9, wherein the auxiliary-indication creating portion creates, as an auxiliary indication, an indication showing a measured value of biometric information of the biological object that serves as the imaging subject, also sets at least two degrees of urgency in accordance with the magnitude of the measured value of the biometric information, creates the auxiliary indication in a normal indication form when the measured value corresponds to the lower degree of urgency, and creates the auxiliary indication in an indication form having a greater emphasis than the normal indication form when the measured value corresponds to the higher degree of urgency.

11. The image-processing device according to Claim 10, wherein, with regard to the auxiliary indication created in the emphasized indication form, the auxiliary-indication creating portion changes the indication form to the normal indication form when the auxiliary indication matches the attention point detected by the attention-point detecting portion.

12. The image-processing device according to any one of Claims 1 to 11, wherein the attention-point detecting portion obtains a track of the attention point by storing the position of the detected attention point over time, and
the auxiliary-indication creating portion creates, as the auxiliary indication, a local-information indication including information about a specific position in the imaging subject, displays the auxiliary indication on the display portion, and moves the auxiliary indication in the direction of the plane based on the track of the attention point obtained by the attention-point detecting portion.

13. The image-processing device according to any one of Claims 1 to 12, wherein the auxiliary-indication creating portion creates, as the auxiliary indication, a local-information indication including information about a specific position in the imaging subject and an arrow that points to the specific position from the local-information indication, assigns the same parallax as a parallax at the attention point to the local-information indication and a base of the arrow, and assigns the same parallax as a parallax at the specific position to a tip of the arrow.

14. A three-dimensional-image observation system comprising:
an image-acquisition device that obtains two parallax images by acquiring images of an imaging subject; and
the image-processing device according to any one of Claims 1 to 13.

15. A three-dimensional-image observation system comprising:
an image-acquisition device that obtains two parallax images by acquiring images of an imaging subject; and
the image-processing device according to Claim 3,
wherein the image-acquisition device obtains, as the parallax images, a normal image acquired by irradiating the imaging subject with illumination light and a fluorescence image obtained by acquiring fluorescence from a fluorescent substance provided in the target object by irradiating the imaging subject with excitation light, and
the attention-point detecting portion detects, as the target object, a fluorescence region in the fluorescence image.
